# EUROPEAN PATENT SPECIFICATION

(11) **EP 3 045 911 B1**
(45) Date of publication and mention of the grant of the patent: **05.06.2019**
(21) Application number: 14844039.9
(22) Date of filing: 05.09.2014
(51) Int. Cl.: G01N 33/48, G01N 37/00, B65B 47/02, B65B 47/06, B65B 61/06, B65B 9/04

(54) **APPARATUS FOR MANUFACTURING DIAGNOSIS KIT**
VORRICHTUNG ZUR HERSTELLUNG EINES DIAGNOSEKITS
APPAREIL DE FABRICATION DE KIT DE DIAGNOSTIC

(30) Priority: 12.09.2013 KR 20130109951
(43) Date of publication of application: 20.07.2016
(73) Proprietor: Access Bio, Inc., Somerset, NJ 08873 (US)
(72) Inventor: YU, Seung Kook, Seoul 158-772 (KR)
(74) Representative: Herrero & Asociados, S.L.
(86) International application number: PCT/KR2014/008370
(87) International publication number: WO 2015/037884

(56) References cited:
- EP-A1- 0 650 904
- WO-A2-2004/054886
- DE-A1- 2 337 730
- DE-U1-202010 010 834
- KR-A- 20070 070 396
- KR-A- 20090 058 727
- KR-A- 20090 101 823
- KR-A- 20110 059 160
- KR-A- 20120 072 832
- KR-A- 20120 072 837
- US-A- 4 034 536
- US-A- 5 729 963
- US-B1- 6 964 145

## Description

### TECHNICAL FIELD

The present invention relates to an apparatus for manufacturing a diagnosis kit, and more particularly, an apparatus for manufacturing a diagnosis kit embedded with a diagnosis strip in a light-weight package, suitably configured for mass production, and a diagnosis kit manufactured thereby.

### BACKGROUND ART

A diagnosis kit is an apparatus to test or examine the existence of a single or a plurality of substances in a liquid sample, for example, a urine or blood sample.

More specifically, the modern diagnostic industry is falling into one type, POCT (Point-Of-Care Testing: POCT).

POCT is a test conducted outside of test laboratories and is a device that can be used by an ordinary person without professional knowledge. Presently, the use of POCT is spreading from hospitals to diagnostic usage in the fields and by individuals.

In particular, rapid diagnostic test kit, which is represented by immunochromatographic analysis, is used in healthcare and medical fields to identify diseases or changes, and various similar devices have been developed in various areas such as food and biological processes, environment technologies, etc., for quantitative and qualitative microanalysis. Its scope of application in the health care is also expanding such as into the field of pregnancy, ovulation, infectious diseases, drug abuse, acute myocardial infarction, and cancer.

Generally, such a diagnosis kit is formed of a case comprising a diagnostic strip according to the target diagnosis.

Here, the case of the diagnosis kit is formed by molding with a certain thickness.

These diagnosis kits are disposable because the diagnostic strip cannot be reused.

As such, the one-time use, disposable diagnosis kits manufactured by plastic molding result in waste of valuable resources.

In addition, manufacturing by molding also leads to increased volume of the diagnosis kits, which in turn require higher logistic cost for transportation.

As a prior reference related to the present invention, the Korean Patent Application Publication No. 10-2012-0086985(published on Aug. 06, 2012) discloses a technology related to a method for manufacturing a diagnosis kit. Patent application EP0650904 describes a number of sub-assemblies for providing an upper film and a lower film, forming cavities in films, placing the item in one of the cavities, sealing the films together along the periphery of the cavities to form a continuous web and cutting the continuous web into strips having the desired number of packages formed therein. EP0650904 discloses an apparatus suitable for manufacturing a diagnosis kit, comprising: a molding material feeding unit for feeding upper molding material and lower molding material via independent feeding paths; wherein the upper molding material and the lower molding material are substantially a sheet or film material, an upper molding unit for receiving the upper molding material from the molding material feeding unit to mold it in a predetermined shape; a lower molding unit for receiving the lower molding material from the molding material feeding unit to mold it in a predetermined shape; a bonding unit for receiving the upper molded material from the upper molding unit and the lower molded material from the lower molding unit and bonding them; and a cutting unit.

### DETAILED DESCRIPTION OF THE INVENTION

### TECHNICAL OBJECTIONS

An aspect of the present invention is to provide an apparatus for manufacturing a diagnosis kit, in a large quantity, which can mold light-weight sheet or film containing a diagnostic strip inside of the sheet of film, and a diagnosis kit manufactured therewith.

Another aspect of the present invention is to provide an apparatus for manufacturing a diagnosis kit which can reduce the weight of the diagnosis kit and a diagnosis kit manufactured thereby.

Still another aspect of the present invention is to provide an apparatus for manufacturing a diagnosis kit, the apparatus can reduce the cost of manufacturing the diagnosis kit, facilitate design diversification, reduce the time period required for the development and preparation for mass production and cost of such development, and a diagnosis kit manufactured thereby.

### MEANS FOR ACHIEVING THE TECHNICAL OBJECTION

The present invention provides an apparatus for manufacturing a diagnosis kit, comprising a molding material feeding unit for feeding upper molding material and lower molding material via independent feeding paths; wherein the upper molding material and the lower molding material are substantially a sheet or film material; and an upper molding unit for receiving the upper molding material from the molding material feeding unit to mold it into an upper diagnosis kit body in a predetermined shape. The upper molding unit comprises an upper heating device for heating the upper molding material fed from the molding material feeding unit to make it moldable; an upper press molding device for press molding the upper molding material being heated and moldable into an upper diagnosis kit body, an upper cooling device for cooling the press-molded upper molding material; and a punching device for punching an air hole in the upper diagnosis kit body.

The upper diagnosis kit body comprises an upper air tube formed in a rectangular circumference shape protruding upwards, a circular tube formed inside of the upper air tube in a circular circumference shape protruding upwards a test reagent injection hole formed inside of the circular tube; and a plurality of air holes formed inside of the air tube at a certain distance from the circular tube, wherein the upper press molding device press molds the upper air tube and the circular tube, and the punching device is provided with plurality of punchers to form punches the test reagent injection hole and the plurality of air holes at the same step.

The apparatus also comprises a lower molding unit for receiving the lower molding material from the molding material feeding unit to mold it into a lower diagnosis kit body in a predetermined shape, and inserting a diagnostic strip in the lower diagnosis kit body. The lower molding unit comprises: a lower heating device for heating the lower molding material fed from the molding material feeding unit to make it moldable; a lower press molding device for press molding the lower molding material heated and moldable into a lower diagnosis kit body; a lower cooling device for cooling the press-molded lower molding material; and a diagnostic strip inserting device form inserting diagnostic strip in the lower diagnosis kit body. The lower diagnosis kit body is formed with a lower air tube having a size corresponding with the size of the upper air tube, and a recessed hole formed inside of the lower air tube by a certain depth for receiving the diagnostic strip, and the lower press molding device forms the lower air tube and the recessed hole by press molding.

The apparatus further comprises a bonding unit for receiving the upper molded material from the upper molding unit and the lower molded material from the lower molding unit and bonding them so that the upper diagnosis kit body and the lower diagnosis kit body are bonded with each other; and a cutting unit for cutting the bonded upper diagnosis kit body and lower diagnosis kit body into a diagnosis kit having a predetermined size.

The upper molding unit is comprised of an upper heating device for heating the upper molding material delivered from the upper material feeder, an upper press molding device for press molding the heated upper molding material into a predetermined shape of an upper diagnosis kit body, an upper cooling device for cooling down the press-molded upper molding material, and a punching device for forming a plurality of air holes in the diagnosis kit body included in the upper molding material.

The upper diagnosis kit body comprises an upper air tube protruding upwards in a rectangular circumference shape, a circular tube protruding upwards in a circular shape placed inside of the upper air tube, a test reagent injection hole formed inside of the circular tube, and a plurality of air holes formed inside of the air tube, with a certain distance from the circular tube.

The upper press molding device press molds the upper air tube and the circular tube, and the punching device preferable punches the test reagent injection hole and the plurality of air holes at the same time.

The lower molding unit is comprised of a lower heating device for heating the lower molding material delivered from the molding material feeder, a lower press molding device for press molding the heated upper molding material into a predetermined shape of a lower diagnosis kit body, a lower cooling device for cooling down the press-molded lower molding material, and a diagnostic strip inserting device for inserting a diagnostic strip in the lower diagnosis kit body.

The lower diagnosis kit body comprises a lower air tube having a size corresponding with that of the upper air tube, and a recessed hole formed inside of the air tube by a predetermined depth for seating the diagnostic strip.

The lower press molding device press-molds the lower air tube and the recess.

In a configuration where the upper diagnosis kit body and the lower diagnosis kit body are formed in plurality,

The diagnostic strip inserting device preferably inserts a plurality of the diagnostic strips delivered in a row in the recessed holes formed in the lower diagnosis kit body sequentially or at the same time.

The bonding unit heats and bonds the upper molded member and the lower molded member.

It is preferable that, with the bottom of the upper diagnosis kit body excluding the upper air tube and the circular tube contacting the upside of the lower diagnosis kit body excluding the lower air tube and the recess, both the two diagnosis kit bodies are heated to be fusion-bonded to form a body of the diagnosis kit.

The cutting unit preferable comprises: a cutting device for cutting out the diagnosis kit body from the fusion-bonded upper and lower molded members; a discharge device provided under the cutting device for dropping and discharging out the cut diagnosis kit body; and a collecting device for collecting the upper and lower molded member from which the diagnosis kit body is cut out.

In a different embodiment, the present invention provides a diagnosis kit manufactured using the apparatus for manufacturing a diagnosis kit in accordance with the present invention.

### Effect of the Invention

The present invention can manufacture diagnosis kits, in large quantities, by press-molding sheet or film containing diagnostic strips inside of the sheet of film.

In addition, the present invention can efficiently prevent deformation of kit itself by comprising a separate air tube on the diagnosis kit comprising a sheet or film material.

In addition, the present invention can reduce the weight of the diagnosis kits.

### Brief Description of the Drawings

FIG. 1 shows an overall configuration in accordance with an embodiment of the present invention.
FIG. 2 is a view of the upper heating device in accordance with an embodiment of the present invention.
FIG. 3 is a perspective view of the upper diagnosis kit body in accordance with an embodiment of the present invention.
FIG. 4 is a top view of the upper diagnosis kit body in accordance with an embodiment of the present invention.
FIG. 5 is a cross-sectional view of FIG. 4 cut along the line A-A.
FIG. 6 is a view of the upper press molding device in accordance with an embodiment of the present invention.
FIG. 7 is a perspective view of the lower diagnosis kit body in accordance with an embodiment of the present invention.
FIG. 8 is a view of the lower press molding device in accordance with an embodiment of the present invention.
FIG. 9 is a cross-sectional view of FIG. 8 cut along the line B-B.
FIG. 10 is a view of the lower press molding device in accordance with an embodiment of the present invention.
FIG. 11 is a view of the diagnostic strip inserting device by a finger insertion type.
FIG. 12 is a view of the diagnostic strip inserting device by a vacuum gripper type,
FIG. 13 is a view of the diagnosis kit in accordance with an embodiment of the present invention, and
FIG. 14 is a cross-sectional view of the diagnosis kit of FIG. 13.

### The Best Mode for the Practice of the Invention

The manufacturing apparatus for diagnosis kit of the present invention is described below referring to the accompanying drawings.

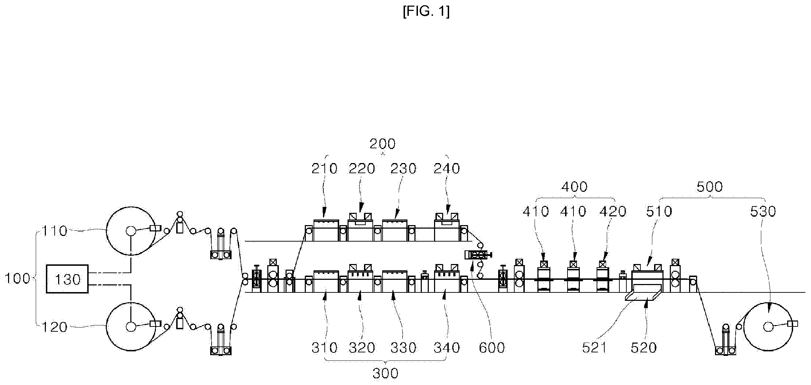

FIG. 1 shows an overall configuration in accordance with an embodiment of the present invention.

Referring to FIG. 1, the apparatus for manufacturing diagnosis kits in accordance with an embodiment of the present invention comprises a molding material feeding unit, an upper molding unit, a lower molding unit, a bonding unit, and a cutting unit.

### Molding Material Feeding Unit (100)

In this description, the upper molding material (1) and the lower molding material (2) are supplied in a roll as a typical embodiment.

In addition, it will be obvious that the upper molding material (1) and the lower molding material (2) can also be supplied in a flat plate or slat form.

The molding material feeding unit (100) may comprise a first feed roll (11), a second feed roll (120), and a rotating device (130) e.g., an electric motor for driving the first and second feed rolls (110, 120).

The upper molding material (1) is wound on the first feed roll (110) and the lower molding material (2) is wound on the second feed roll (12).

Here, the upper molding material (1) and lower molding material (2) are formed in a sheet or film shape.

The upper and lower molding materials (1) and (2) can be any one of PA, APET, COPET, PET, PE, ABS, PS, PP, CPR, EG, PVC, PC, EVA, LDPE, and EVOH or a combination thereof.

In addition, the upper and lower molding materials (1) and (2) can be any material which can be heated and press molded.

A first feed path is provided for the upper molding material (1) between the first feed roll (110) and upper molding unit (200).

A second feed path is provided for the lower molding material (2) between the second feed roll (120) and lower molding unit (300).

A plurality of dancer rolls (D) are installed in the first feed path and the second feed path. The dancer rolls (D) apply appropriate tension to the upper and lower molding materials (1) and (2) to ensure stable moving along the first feed path and the second feed path.

Meanwhile, the upper molding material (1) and lower molding material (2) may be applied with an additional window forming process (not shown in the drawings) before they are wound on the first and second feed rolls (110) and (120).

The window is formed with a transparent material. The entire upper molding material (1) and lower molding material (2) can be formed with a transparent material or only a portion of the materials are provided with windows made of a transparent material.

### Upper Molding Unit (200)

The upper molding unit (200) comprises an upper heating device (210), and upper press molding device (220), an upper cooling device (230), and a punching device (240).

The upper molding unit (200) provides a first molding path in connection with the first feed path.

In addition, the upper heating device (210), upper press molding device (220), upper cooling device (230), and punching device (240) are arranged along in the first molding path in said order.

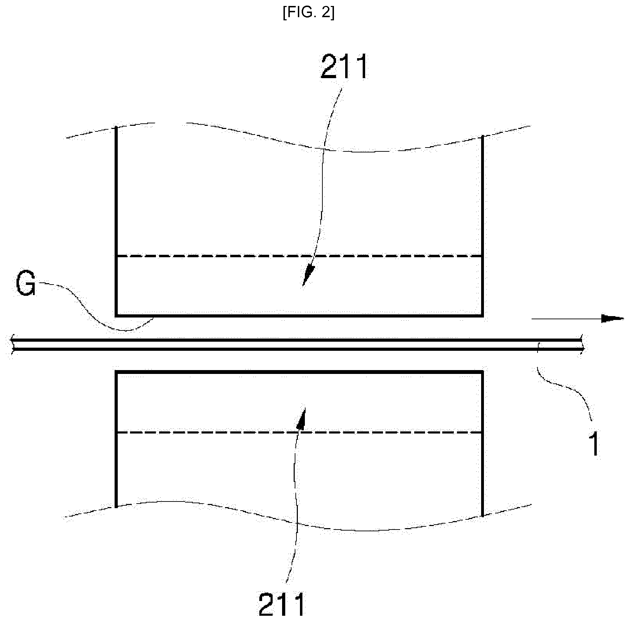

FIG. 2 is a view of the upper heating device in accordance with an embodiment of the present invention.

Referring to FIG. 2, the upper heating device (210) comprises a heating block (211) provided with a gap (G) through which the upper molding material (1) passes.

The heating block (211) is provided with a heater device (not shown) connected to an outside power source.

As such, the upper molding material (1) is heated by the heater device up to a predetermined temperature passing through the gap (G) of the heating block (211).

By the heating, the upper molding material (1) is changed to a press-moldable state.

In the present invention, the heating method can be a high frequency induction heating, radiative heating, or hot air heating.

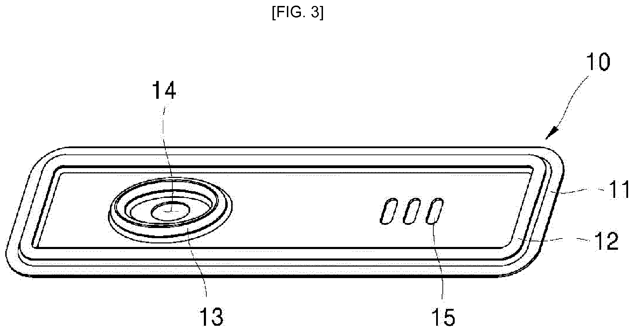

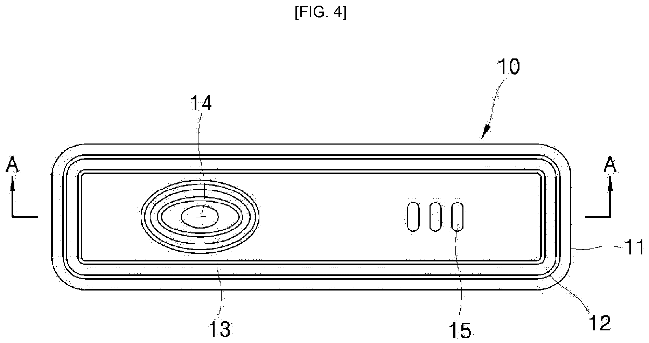

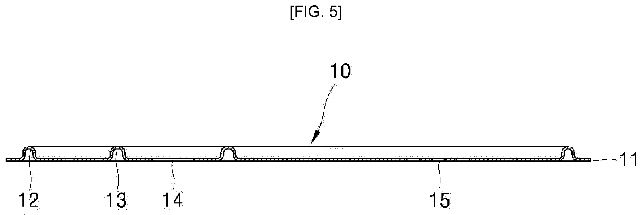

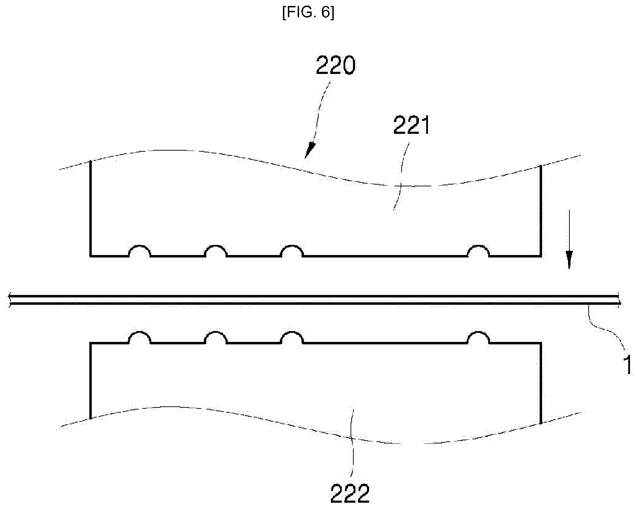

FIG. 2 is a view of the upper heating device in accordance with an embodiment of the present invention, FIG. 3 is a perspective view of the upper diagnosis kit body in accordance with an embodiment of the present invention, FIG. 4 is a top view of the upper diagnosis kit body in accordance with an embodiment of the present invention, FIG. 5 is a cross-sectional view of FIG. 4 cut along the line A-A, and FIG. 6 is a view of the upper press molding device in accordance with an embodiment of the present invention.

Next to the upper heating device (210) is an upper press molding device (220).

The upper press molding device (220) is a press molding unit.

Referring to FIGs. 3 to 5, the configuration of the upper diagnosis kit body (10) in accordance with an embodiment of the present invention is described.

The upper diagnosis kit body (10) comprises: an upper body (11) formed with the upper molding material (1), un upper air tube (12) forming a rectangular circumference protruding upwards on the upper body (11), and a circular tube provided inside wall of the upper air tube (12).

In addition, a test reagent injection through-hole (14) is formed inside of the circular tube, and a plurality of air holes (15) are formed inside of the upper air tube (12) at a certain distance from the circular tube.

Referring to above described configuration, the upper press molding device (220) can comprise an upper mold (221) and a lower mold (222),and the upper molding material (1) is pressed and molded between the upper mold (221) and lower mold (222).

For example, referring to FIG. 6, the upper mold (221) is formed with the engraved patterns corresponding with the shapes of the upper air tube (12) and circular tube (13), and the lower mold (222) is formed with embossed patterns corresponding with the shapes of the upper air tube (12) and circular tube (13).

Accordingly, the upper molding material (1) is placed between the upper mold (221) and lower mold (222), and the upper mold (221) and lower mold (222) are pressed together to press-mold the upper molding material (1).

In addition, the test reagent injection hole (14) and punch holes (15) are formed with a separate punching device (240).

Here, any one or both of the upper mold (221) and lower mold (222) are provided with additional heating members (not shown), and any one or both of the upper mold (221) and lower mold (222) heated up by the heating members.

The upper cooling device (230) cools down the upper molding material (1) which is transferred after being formed with the upper air tube (12) and circular tube (13) by press molding described above, to a predetermined temperature level.

The cooling method can be water cooling or air cooling.

If an air cooling device is used, it is preferable to blow low cooling air on one side of the upper molding material (1) at a uniform pressure.

The punching device (240) is provided with a puncher (not shown) which moves up and down to punch holes.

Here, a plurality of punchers are provided to form test reagent injection hole (14) and air holes (15).

After cooling, the upper molding material (1) is fed into and placed at a predetermined position in the punching device (240).

The punch heads moves up and down to punch the test reagent injection hole (14) and air holes (15) in the upper molding material (1) placed at the predetermined position.

It should be noted that, the punching device (240) is separated from the upper cooling device (230) by a predetermined distance. The spacing allows the upper molding material (1) to be cooled down close to the room temperature so that the rims of the punch holes are not damaged.

Passing through the punching device (240), the upper molding material (1) is transferred to the arranging unit (600).

The method of preheating for heat forming can make use of hot air blow, a heating block, or a heating roll.

### Lower Molding Unit (300)

The lower molding unit (300) comprises a lower heating device (310), a lower press molding device (320), a lower cooling device (330), and a diagnostic strip inserting device (340).

The lower molding unit (300) provides a second molding path in connection with the second feed path.

In addition, the lower heating device (310), lower press molding device (320), lower cooling device (330), and diagnostic strip inserting device (340) are arranged along in the second molding path in said order.

The lower heating device (310) comprises a heating block (311) provided with a gap (G) through which the lower molding material (2) passes.

The heating block (311) is provided with a heating member connected to an outside power source.

As such, the lower molding material (2) is heated by the heating member up to a predetermined temperature passing through the gap (G) of the heating block (311).

By the heating, the lower molding material (2) is changed to a press-moldable state.

In the present invention, the heating method can be a high frequency induction heating, radiative heating, or hot air heating.

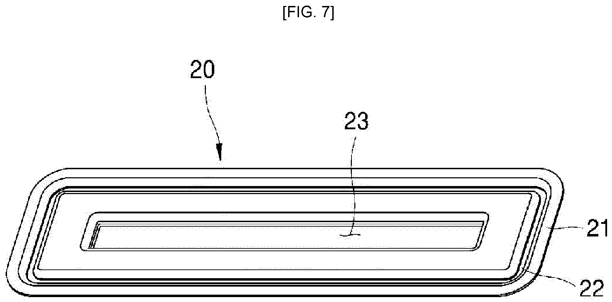

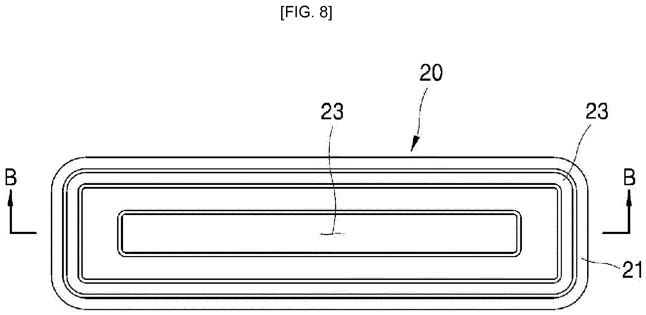

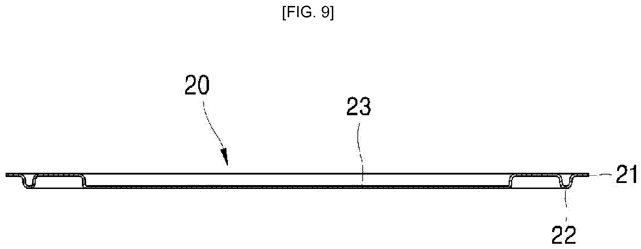

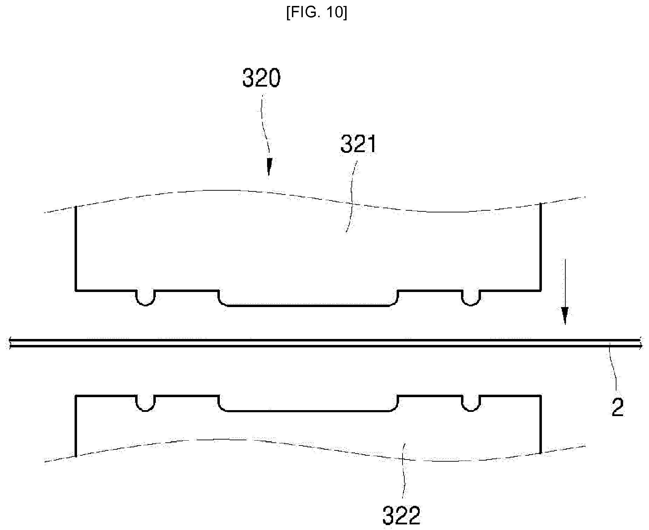

FIG. 7 is a perspective view of the lower diagnosis kit body in accordance with an embodiment of the present invention, FIG. 8 is a top view of the lower molding device in accordance with an embodiment of the present invention, FIG. 9 is a cross-sectional view of FIG. 8 cut along the line B-B, and FIG. 10 is a view of the lower press molding device in accordance with an embodiment of the present invention,

Next to the lower heating device (310) is a lower press molding device (320).

The lower press molding device (320) can be a press molding unit.

The configuration of a lower diagnosis kit body (20) in accordance with an embodiment of the present invention is described below.

The lower diagnosis kit body (20) comprises: a lower body (21) formed with the lower molding material (2), a lower body (20) convex downwards forming a rectangular circumference on the lower body (21), and a recessed hole (23) formed by a certain depth inside of the lower air tube (22).

Referring to above described configuration, the lower press molding device (320) can comprise an upper mold (321) and a lower mold (322),and the lower molding material (2) is pressed and molded between the upper mold (321) and lower mold (322).

For example, referring to FIGs. 7 to 9, the upper mold (321) is formed with embossed patterns corresponding with the shapes of the lower air tube (22) and recessed hole (23), and the lower mold is formed with engraved patterns corresponding with the shapes of the lower air tube (22) and recessed hole (23).

Accordingly, the lower molding material (2) is placed between the upper mold (321) and lower mold (322), and the upper mold (321) and lower mold (322) are pressed together to press-mold the lower molding material (2).

Here, the upper molding material (1) and lower molding material (2) can be formed with a plurality of upper diagnosis kit bodies (10) and lower diagnosis kit bodies (20) at a certain intervals.

Here, any one or both of the upper mold (221) and lower mold (222) are provided with additional heating members (not shown), and any one or both of the upper mold (221) and lower mold (222) heated up by the heating members.

The lower cooling device (330) cools down the lower molding material (2) which is transferred after being press-formed with the lower air tube (22) and recessed hole (23) described above, to a predetermined temperature level.

The cooling method can be water cooling or air cooling.

If an air cooling device is used, it is preferable to blow low cooling air on one side of the upper molding material (1) at a uniform pressure.

The diagnostic strip inserting device (340) inserts a diagnostic strip (30) in the recessed hole (23) formed in the lower diagnosis kit body (20) which has been cooled down.

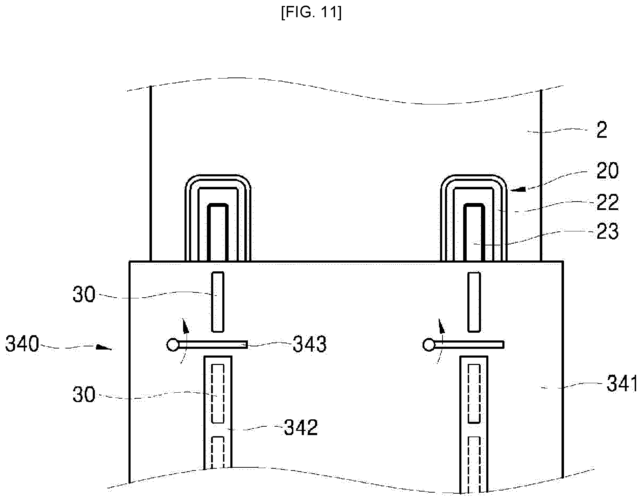

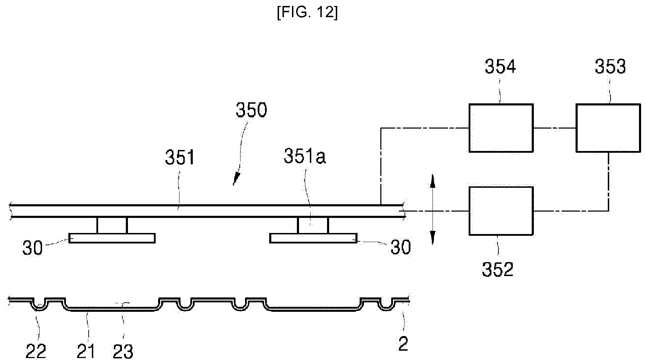

Diverse exemplary methods of inserting diagnostic strips in accordance with the present invention are described by referring to FIGs. 11 and 12.

FIG. 11 is a view of the diagnostic strip inserting device by a finger insertion type.

Referring to FIG. 11, the diagnostic strip inserting device (340) comprises: a strip loading device (342) which sequentially feeds diagnostic strips (30) to the base (341) and the feeding position formed on the base (341); and a motored swivel finger (343) which is provided on the lateral side of the feeding position and pushes the diagnostic strips (30) placed in the feeding position into the recessed hole (23).

Here, the base (341) is preferably positioned at a higher level than the second forming path.

Accordingly, when the press molded and cooled lower molding material (2) is transferred to the underside of the base (341), the swivel finger (343) rotates and pushes the diagnostic strip (30) placed at the feeding position into the recessed hole (22).

FIG. 12 is a view of a vacuum gripper type diagnostic strip inserting device.

Referring to FIG. 12, the diagnostic strip inserting device (350) comprises: a suction plate (351) having suction members (351a) for gripping a plurality of the diagnostic strips (30); a displacing device (352) for displacing the suction plate (351); and a vacuum controller (353) for generating vacuum pressure and controlling gripping.

Here, the spacing between the suction members (351a) shall be substantially the same as the spacing between the recessed holes (23) formed on the lower molding material (2).

Accordingly, when the press-molded and cooled lower molding material (2) is transferred to the underside of the base (341), the suction plate (351) moves to the upside of the lower molding material (2) by the displacing device.

Here, the positions of the recessed holes (23) formed on the lower molding material (2) and the suction members (351a) are the same. The suction members (351a) are gripping the diagnosis kits by vacuum pressure.

The suction plate (351) is lowered by the displacing device (352) so that the diagnostic strips (30) are seated in the recessed holes (23), and the vacuum controller releases the vacuum pressure applied to the suction members (351a).

Accordingly, the vacuum strips (30) can be seated in the recessed holes (23).

While the method of inserting the diagnostic strips (30) was described above according to a typical method, any other methods which can seat the diagnostic strips (30) in the recessed holes (23) are applicable.

As described above, the lower molded material (2) seated with the diagnostic strips (30) is transferred to the arranging unit (600).

The preheating can be achieved by uisng hot air blow, a heating block, or a heating roll.

According to invention, the upper molding material and the lower molding material are press molded, however, in an exemplary embodiment which is not in the scope of the invention, the upper molding material and the lower molding material can also be vacuum molded.

That is, while not depicted in the drawings, by contacting a body formed with desired patterns to the upper or lower surface of the preheated and moldable upper molding material and applying vacuum pressure to the desired patterns using an external vacuum suction device, the upper molding material can be vacuum molded in a desired shape.

In an exemplary embodiment which is not in the scope of the invention, the lower molding material can also be molded with the same vacuum molding method as the upper molding material.

### Arranging Unit (600)

The arranging unit (600) in accordance with an embodiment of the present invention arranges the upper and lower molded materials (1) and (2) moving along the first and second molding paths, respectively, can be matched at a predetermined positions.

That is, it is preferable that the arrangement state shall be so that the upper air tube (12) formed on the upper molding material (1) and the lower air tube (22) formed on the lower molding material (2) are facing each other.

### Bonding Unit (400)

Referring to FIG. 1, the bonding unit (400) in accordance with an embodiment of the present invention fusion-bonds the upper and lower molding materials (1) and (2) having passed the arranging unit (600) and contacting each other.

The bonding unit (400) provides a bonding path through which the contacting upper and lower molding materials (1) and (2) pass.

The bonding unit (400) comprises one or more heat bonding devices (410) and a cooling device which are arranged on the bonding path sequentially.

The one or more heat bonding devices (410) fusion-bonds the contacting surfaces of the upper and lower molding materials (1) and (2), sequentilly, excluding the upper and lower air tubes (12) and (22).

The one or more heat bonding devices (410) can be ultrasonic welding devices, and their operation can be integrated into one step according to the dimension of the diagnosis kit to be manufactured.

In addition, the bonded material (1') can be bonded by ultrasonic fusion.

The cooling device (420) cools down the bonded upper and lower molding members (hereinafter, "bonded material").

Here, the bonded material (1') is formed with a plurality of diagnosis kits.

Then, the cooled bonded material (1') is transferred to the cuting unit (500).

In an embodiment of the present invention, the heat bonding device can use any one of heat fusion, high frequency induction fusion, and ultrasonic fusion.

### Cutting Unit (500)

The cutting unit (500) cuts out the diagnosis kits from the bonding unit (400).

The cutting unit (500) is provided with a cutting device (510) having cutter knives (not shown) arranged corresponding with the circumference of the diagnosis kit, and cuts the circumference by moving up and down.

In addition, if the bonded material (1') is formed with a plurality of diagnosis kits at a certain intervals, the cutting unit (500) may be provided with the cutter knives whose number corresponds with the number of the diagnosis kits.

Accordingly, a plurality of the diagnosis kits can be cut off at once by the cutter knives moving up and down.

The cutting unit (500) is provided with a discharge device (520) under the cutting device (510) for dropping and discharging the cutpoff diagnosis kits.

The discharge device (520) is provided under the cutting device (510) and provided with a guide plate (521) for guiding the falling diagnosis kits.

In addition, the cutting unit (500) is provided with a collecting device (530) for collecting the bonded material (1') excluding the diagnosis kits (hereinafter, "scrap").

The collecting device (530) can be a collecting roller which winds and collects the bonded material (1') from which the diagnosis kits have been cut off.

In the present invention, the scraps can be cut and provessed.

The apparatus for manufacturing the diagnosis kits in accordance with an embodiment of the present invention is described above.

### Embodiments of the Present Invention

The diagnosis kit manufactured with the apparatus for manufacturing the diagnosis kits in accordance with an embodiment of the present invention is described in detail below.

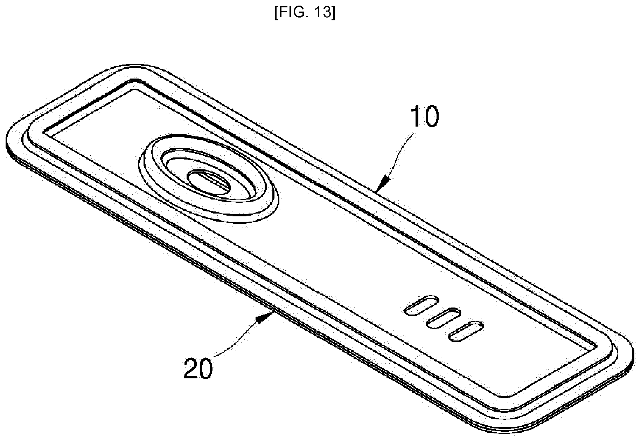

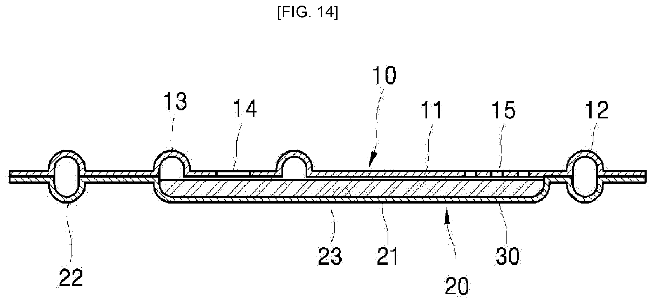

FIG. 13 is a view of the diagnosis kit in accordance with an embodiment of the present invention, and FIG. 14 is a cross-sectional view of the diagnosis kit of FIG. 13.

The configuration of the upper diagnosis kit body (10) and lower diagnosis kit body (20) will refer to FIGs. 3 and 7.

Referring to FIG. 13 and 14, the diagnosis kit in accordance with an embodiment of the present invention comprises an upper diagnosis kit body (10) and a lower diagnosis kit body (20) which are fusion bonded with each other.

### Upper Diagnosis Kit Body

The upper diagnosis kit body (10) is formed from a sheet or film material and provided with an upper body (11) which is a rectangular plate. It would be obvious for those skilled in the art that the the upper diagnosis kit body (10) can be formed in various shapes.

The upper body (11) is formed with an upper air tube (12) and a circular tube (13).

The upper air tube (12) forms a rectangular circumference on the upper body (11) and protrudes upward.

Under the upper body (11), the upper air tube (12) is formed with a space inside.

A purpose of the upper air tube (12) is to prevent the warping of the upper body (11).

Here, on the upper body (11), a circumference having a certain width is formed outside of the upper air tube (12).

In addition, the circular tube (13) is formed inside of the upper air tube (12) protruding upwards from the upper body (11).

Here, the longitudinal cross sections of the the upper air tube (12) and circular tube (13) can be the same.

The circular tube (13) also provides a support.

Furthermore, a test reagent injection hole (14) which is a vertical through-hole is formed inside of the circular tube (13).

And, a plurality of the air holes (15) are formed inside of the upper air tube (12) at a certain distance from the circular tube (13).

The plurality of air holes (15) are rectangular holes and formed as through-holes of the upper body (11).

Here, the formation of the upper diagnosis kit body (10) is described in the description of the apparatus for manufacturing, thus, not described here.

In addition, the upper air tube (12) and circular tube (13) can be formed in various shapes.

### Lower Diagnosis Kit Body

The lower diagnosis kit body (20) is formed from a sheet or film material and provided with a lower body (21) formed in a rectangular planar-shape.

The lower body (21) is formed with an lower air tube (22) and a recessed hole (23).

The lower air tube (22) is formed in a rectagular circumference shape on the underside of the lower body (21) protruding downwards.

Here, the shape of the lower air tube (22) may be substantially the same as that of the upper air tube (12).

The recessed hole (23) is provided with a step or steps (s) at a certain depth on the upper surface of the lower body (21).

The size of the recessed hole (23) may be determined variably in accordance with the size of the diagnostic strip (30) to be seated in the recess.

Here, since the description of the formation of the lower diagnosis kit body (20) is described in the description of the apparatus for manufacturing, thus, omitted.

In accordance with an embodiment of the present invention, the upper diagnosis kit body (10) and the lower diagnosis kit body (20) are contacted and bonded with each other, and the method of bonding is preferably heat fusion as described above.

That is, because the upper and lower diagnosis kit bodies (10 and 20) are formed from a sheet or film material, they can be fusion bonded.

Accordingly, the upper body (11) and the lower body (21) form an integral body and the upper and lower air tubes are formed protruding upwards and downwards, respectively.

In addition, the lower air tube (22) can be formed in various shapes.

And, top of the recessed hole (23) having a certain depth and length is covered by the upper body (11).

Here, the recessed hole (23) is seated with a diagnostic strip (30).

The diagnostic strip (30) is a test material that can diagnose various diseases.

Here, ann end of the diagnostic strip (30) is exposed to the test reagent injection hole (14) formed in the upper body (11) while another end is exposed to the plurality of air holes (15).

As such, an end of the diagnostic strip is smeared with the test reagent entering through the test reagent injection hole, and the test reagent can be easily transferred to the opposite end by the air penetrating into the air holes on the opposite end.

The exemplary embodiments of the apparatus for manufacturing a diagnosis kit and the diagnosis kit manufactured thereby in accordance with the present invention are described above, it would be obvious that various modifications and changes can be made without departing from the scope of the present invention.

The present invention may be embodied in other specific forms without departing from the essential attributes thereof and accordingly, reference should be made to the appended claims rather than to the foregoing specification as indicating the scope of the invention

Therefore, the embodiments described here shall be interpreted to be exemplary and not limiting the present invention. It will be understood that the appended claims are intended to cover all such modifications and embodiments which come within the scope of the present invention.

## Claims

1. An apparatus for manufacturing a diagnosis kit, comprising:
a molding material feeding unit for feeding upper molding material (1) and lower molding material (2) via independent feeding paths; wherein the upper molding material and the lower molding material are substantially a sheet or film material;
an upper molding unit (200) for receiving the upper molding material (1) from the molding material feeding unit to mold it into an upper diagnosis kit body (10) in a predetermined shape;
wherein the upper molding unit comprises
an upper heating device (210) for heating the upper molding material fed from the molding material feeding unit to make it moldable;
an upper press molding device (220) for press molding the upper molding material heated and moldable into an upper diagnosis kit body;
an upper cooling device (230) for cooling the press-molded upper molding material; and
a punching device (240) for punching an air hole in the upper diagnosis kit body (10);
wherein the upper diagnosis kit body (10) comprises:
an upper air tube (12) formed in a rectangular circumference shape protruding upwards;
a circular tube (13) formed inside of the upper air tube in a circular circumference shape protruding upwards;
a test reagent injection hole (14) formed inside of the circular tube; and
a plurality of air holes (15) formed inside of the air tube at a certain distance from the circular tube,
wherein the upper press molding device press molds the upper air tube and the circular tube, and the punching device (240) is provided with plurality of punchers to form the test reagent injection hole and the plurality of air holes ;
a lower molding unit (300) for receiving the lower molding material from the molding material feeding unit to mold it into a lower diagnosis kit body in a predetermined shape, and inserting a diagnostic strip in the lower diagnosis kit body;
wherein the lower molding unit comprises:
a lower heating device (310) for heating the lower molding material fed from the molding material feeding unit to make it moldable;
a lower press molding device (320) for press molding the lower molding material heated and moldable into a lower diagnosis kit body;
a lower cooling device (330) for cooling the press-molded lower molding material; and
a diagnostic strip inserting device (340) for inserting a diagnostic strip in the lower diagnosis kit body;
wherein the lower diagnosis kit body is formed with a lower air tube having a size corresponding with the size of the upper air tube, and a recessed hole formed inside of the lower air tube by a certain depth for receiving the diagnostic strip, and the lower press molding device forms the lower air tube and the recessed hole by press molding;
a bonding unit (400) for receiving the upper molded material from the upper molding unit and the lower molded material from the lower molding unit and bonding them so that the upper diagnosis kit body and the lower diagnosis kit body are bonded with each other; and
a cutting unit (500) for cutting the bonded upper diagnosis kit body and lower diagnosis kit body into a diagnosis kit having a predetermined size.

2. The apparatus of claim 1, if the upper diagnosis kit body and the lower diagnosis kit body are formed in a plurality, the diagnostic strip inserting device inserts the diagnostic strips in place into the recessed holes formed in the lower diagnosis kit body sequentially or all at once.

3. The apparatus of claim 1, wherein the bonding unit receives the upper molded material and the lower molded material by heat fusion bonding or ultrasonic fusion bonding, contacting the bottom of the upper diagnosis kit body excluding the upper air tube and the circular tube and the upside of the lower diagnosis kit body excluding the lower air tube and the recessed holes, and bonding the contact surface by heating to form a diagnosis kit body.

4. The apparatus of claim 3, wherein the cutting unit comprises:
a cutting device (510) for cutting out the diagnosis kit body from the bonded upper and lower molded materials;
a discharging device (520) for dropping and discharging the cut-out diagnosis kit body; and
a collecting device (530) for collecting the upper and lower molded materials from which the diagnosis kit has been cut off.

## Patentansprüche

1. Vorrichtung zur Herstellung eines Diagnosekits, umfassend:
eine Formmaterialzuführeinheit zum Zuführen von oberem Formmaterial (1) und unterem Formmaterial (2) über unabhängige Zuführwege; wobei das obere Formmaterial und das untere Formmaterial im Wesentlichen ein Blatt- oder Folienmaterial sind; eine obere Formeinheit (200) zum Aufnehmen des oberen Formmaterials (1) von der Formmaterialzuführeinheit, um es zu einem oberen Diagnosekitkörper (10) in einer vorbestimmten Form zu formen;
wobei die obere Formeinheit Folgendes umfasst:
eine obere Heizeinrichtung (210) zum Erwärmen des oberen von der Formmaterialzuführeinheit zugeführten Formmaterials, um es formbar zu machen; eine obere Pressformeinrichtung (220) zum Pressformen des oberen Formmaterials, das erwärmt und zu einem oberen Diagnosekitkörper formbar wurde; eine obere Kühleinrichtung (230) zum Kühlen des pressgeformten oberen Formmaterials; und
eine Stanzeinrichtung (240) zum Stanzen eines Luftlochs in den oberen Diagnosekitkörper (10);
wobei der obere Diagnosekitkörper (10) Folgendes umfasst:
ein oberes Luftrohr (12), das in einer rechteckigen Umfangsform ausgebildet ist und nach oben vorsteht;
ein kreisförmiges Rohr (13), das innerhalb des oberen Luftrohrs in einer kreisförmigen Umfangsform ausgebildet ist und nach oben vorsteht;
ein Testreagensinjektionsloch (14), das innerhalb des kreisförmigen Rohrs ausgebildet ist; und eine Mehrzahl von Luftlöchern (15), die innerhalb des Luftrohrs in einem bestimmten Abstand von dem kreisförmigen Rohr ausgebildet sind,
wobei die obere Pressformeinrichtung das obere Luftrohr und das kreisförmige Rohr pressformt und die Stanzeinrichtung (240) mit einer Mehrzahl von Stanzern versehen ist, um das Testreagensinjektionsloch und die Mehrzahl von Luftlöchern auszubilden;
eine untere Formeinheit (300) zum Aufnehmen des unteren Formmaterials von der Formmaterialzuführeinheit, um es zu einem unteren Diagnosekitkörper in einer vorbestimmten Form zu formen, und Einführen eines Diagnosestreifens in den unteren Diagnosekitkörper;
wobei die untere Formeinheit Folgendes umfasst:
eine untere Heizeinrichtung (310) zum Erwärmen des unteren von der Formmaterialzuführeinheit zugeführten Formmaterials, um es formbar zu machen;
eine untere Pressformeinrichtung (320) zum Pressformen des unteren Formmaterials, das erwärmt und zu einem unteren Diagnosekitkörper formbar wurde;
eine untere Kühleinrichtung (330) zum Kühlen des pressgeformten unteren Formmaterials; und
eine Diagnosestreifeneinführeinrichtung (340) zum Einführen eines Diagnosestreifens in den unteren Diagnosekitkörper;
wobei der untere Diagnosekitkörper mit einem unteren Luftrohr mit einer Größe, die der Größe des oberen Luftrohrs entspricht, und einem versenkten Loch ausgebildet ist, das innerhalb des unteren Luftrohrs um eine bestimmte Tiefe zum Aufnehmen des Diagnosestreifens ausgebildet ist, und die untere Pressformeinrichtung das untere Luftrohr und das versenkte Loch durch Pressformen ausbildet;
eine Verbindungseinheit (400) zum Aufnehmen des oberen Formmaterials von der oberen Formeinheit und des unteren Formmaterials von der unteren Formeinheit und Verbinden derselben, sodass der obere Diagnosekitkörper und der untere Diagnosekitkörper miteinander verbunden sind; und
eine Schneideinheit (500) zum Schneiden des verbundenen oberen Diagnosekitkörpers und unteren Diagnosekitkörpers in ein Diagnosekit mit einer vorbestimmten Größe.

2. Vorrichtung nach Anspruch 1, wobei dann, wenn der obere Diagnosekitkörper und der untere Diagnosekitkörper in einer Mehrzahl ausgebildet sind, die Diagnosestreifeneinführeinrichtung die Diagnosestreifen an Ort und Stelle in die versenkten Löcher einführt, die in dem unteren Diagnosekitkörper nacheinander oder gleichzeitig ausgebildet wurden.

3. Vorrichtung nach Anspruch 1, wobei die Verbindungseinheit das obere Formmaterial und das untere Formmaterial durch Wärmeschmelzverbinden oder Ultraschallschmelzverbinden aufnimmt, den Boden des oberen Diagnosekitkörpers mit Ausnahme des oberen Luftrohrs und des kreisförmigen Rohrs sowie die Oberseite des unteren Diagnosekitkörpers mit Ausnahme des unteren Luftrohrs und der versenkten Löcher kontaktiert und die Kontaktfläche durch Erwärmen verbindet, um einen Diagnosekitkörper auszubilden.

4. Vorrichtung nach Anspruch 3, wobei die Schneideinheit Folgendes umfasst:
eine Schneideinrichtung (510) zum Ausschneiden des Diagnosekitkörpers aus dem verbundenen oberen und unteren Formmaterial;
eine Entladeeinrichtung (520) zum Fallenlassen und Entladen des ausgeschnittenen Diagnosekitkörpers;
und eine Erfassungseinrichtung (530) zum Erfassen des oberen und unteren Formmaterials, aus dem das Diagnosekit ausgeschnitten wurde.

## Revendications

1. Appareil pour fabriquer un kit de diagnostic, comprenant :
une unité d'alimentation en matériau de moulage pour fournir un matériau de moulage supérieur (1) et un matériau de moulage inférieur via des trajets d'alimentation indépendants ; dans lequel le matériau de moulage supérieur et le matériau de moulage inférieur sont essentiellement un matériau de feuille ou de film ;
une unité de moulage supérieure (200) pour recevoir le matériau de moulage supérieur (1) depuis l'unité d'alimentation en matériau de moulage pour le mouler en un corps supérieur de kit de diagnostic (10) sous une forme prédéterminée ;
dans lequel l'unité de moulage supérieure comprend
un dispositif de chauffage supérieur (210) pour chauffer le matériau de moulage supérieur fourni par l'unité d'alimentation en matériau de moulage pour le rendre moulable ;
un dispositif de moulage à la presse supérieur (220) pour mouler à la presse le matériau de moulage supérieur chauffé et moulable en un corps supérieur de kit de diagnostic ;
un dispositif de refroidissement supérieur (230) pour refroidir le matériau de moulage supérieur moulé à la presse ; et
un dispositif de perforation (240) pour perforer un trou d'air dans le corps supérieur de kit de diagnostic (10) ;
dans lequel le corps supérieur de kit de diagnostic (10) comprend :
un tube d'air supérieur (12) formé sous une forme de circonférence rectangulaire faisant saillie vers le haut ;
un tube circulaire (13) formé à l'intérieur du tube d'air supérieur sous une forme de circonférence circulaire faisant saillie vers le haut ;
un trou d'injection de réactif d'essai (14) formé à l'intérieur du tube circulaire ; et
une pluralité de trous d'air (15) formés à l'intérieur du tube d'air à une certaine distance du tube circulaire,
dans lequel le dispositif de moulage à la presse supérieur moule à la presse le tube d'air supérieur et le tube circulaire, et le dispositif de perforation (240) est pourvu d'une pluralité de perforateurs pour former le trou d'injection de réactif d'essai et la pluralité de trous d'air ;
une unité de moulage inférieure (300) pour recevoir le matériau de moulage inférieur depuis l'unité d'alimentation en matériau de moulage pour le mouler en un corps inférieur de kit de diagnostic sous une forme prédéterminée, et insérer une bandelette de diagnostic dans le corps inférieur de kit de diagnostic ;
dans lequel l'unité de moulage inférieure comprend :
un dispositif de chauffage inférieur (310) pour chauffer le matériau de moulage inférieur fourni par l'unité d'alimentation en matériau de moulage pour le rendre moulable ;
un dispositif de moulage à la presse inférieur (320) pour mouler à la presse le matériau de moulage inférieur chauffé et moulable en un corps inférieur de kit de diagnostic ;
un dispositif de refroidissement inférieur (330) pour refroidir le matériau de moulage inférieur moulé à la presse ; et
un dispositif d'insertion de bandelette de diagnostic (340) pour insérer une bandelette de diagnostic dans le corps inférieur de kit de diagnostic ;
dans lequel le corps inférieur de kit de diagnostic est formé avec un tube d'air inférieur ayant une taille correspondant à la taille du tube d'air supérieur, et un trou renfoncé formé à l'intérieur du tube d'air inférieur à une certaine profondeur pour recevoir la bandelette de diagnostic, et le dispositif de moulage à la presse inférieur forme le tube d'air inférieur et le trou renfoncé par moulage à la presse ;
une unité de liaison (400) pour recevoir le matériau moulé supérieur provenant de l'unité de moulage supérieure et le matériau moulé inférieur provenant de l'unité de moulage inférieure et les lier de façon que le corps supérieur de kit de diagnostic et le corps inférieur de kit de diagnostic soient liés l'un à l'autre ; et
une unité de découpe (500) pour couper le corps supérieur de kit de diagnostic et le corps inférieur de kit de diagnostic liés en un kit de diagnostic ayant une taille prédéterminée.

2. Appareil selon la revendication 1, si le corps supérieur de kit de diagnostic et le corps inférieur de kit de diagnostic sont formés en une pluralité, le dispositif d'insertion de bandelette de diagnostic insère les bandelettes de diagnostic en place dans les trous renfoncés formés dans le corps inférieur de kit de diagnostic de manière séquentielle ou toutes en même temps.

3. Appareil selon la revendication 1, dans lequel l'unité de liaison reçoit le matériau moulé supérieur et le matériau moulé inférieur par liaison par fusion thermique ou par liaison par fusion par ultrasons, en mettant en contact la partie inférieure du corps supérieur de kit de diagnostic à l'exclusion du tube d'air supérieur et du tube circulaire et le côté supérieur du corps inférieur de kit de diagnostic à l'exclusion du tube d'air inférieur et des trous renfoncés, et en liant la surface de contact par chauffage pour former un corps de kit de diagnostic.

4. Appareil selon la revendication 3, dans lequel l'unité de découpe comprend :
un dispositif de découpe (510) pour couper le corps de kit de diagnostic à partir des matériaux moulés supérieur et inférieur liés ;
un dispositif de déchargement (520) pour laisser tomber et décharger le corps de kit de diagnostic coupé ; et
un dispositif de collecte (530) pour collecter les matériaux moulés supérieur et inférieur à partir desquels le kit de diagnostic a été coupé.
